# EUROPEAN PATENT SPECIFICATION

(11) **EP 4 218 590 B1**
(45) Date of publication and mention of the grant of the patent: **27.08.2025**
(21) Application number: 21880444.1
(22) Date of filing: 12.10.2021
(51) Int. Cl.: A61B 6/40, A61B 6/03, A61B 6/00

(54) **COMPUTED TOMOGRAPHY DEVICE AND DRIVING METHOD THEREOF**
COMPUTERTOMOGRAFIEVORRICHTUNG UND ANSTEUERUNGSVERFAHREN DAFÜR
DISPOSITIF DE TOMODENSITOMÉTRIE ET SON PROCÉDÉ DE COMMANDE

(30) Priority: 12.10.2020 KR 20200131033
(43) Date of publication of application: 02.08.2023
(73) Proprietor: Sstlabs, Gyeonggi-do 13486 (KR)
(72) Inventor: CHOI, Kwang Yun, Gwangju-si Gyeonggi-do 12736 (KR)
(74) Representative: Stolmár & Partner Patentanwälte PartG mbB
(86) International application number: PCT/KR2021/013980
(87) International publication number: WO 2022/080797

(56) References cited:
- JP-A- 2004 000 356
- JP-A- 2006 520 235
- KR-A- 20200 057 735
- KR-B1- 101 665 327
- KR-B1- 101 665 327
- US-A1- 2005 135 550
- US-A1- 2005 175 143
- BRUDER H. ET AL: "Correction of cross-scatter in next generation dual source CT (DSCT) scanners", PROCEEDINGS OF SPIE, vol. 6913, 6 March 2008 (2008-03-06), pages 69131W, XP093127613, ISSN: 0277-786X, DOI: 10.1117/12.768471

## Description

### 1. Field

The present disclosure relates to a computed tomography (CT) apparatus using x-rays, particularly, to a stationary gantry computed tomography apparatus, and more particularly, to a computed tomography apparatus, where fan beam type multi-sources are spaced along a transfer direction of a test subject, with one pair of adjacent sources having an angle between 90 to 180 degrees, thereby minimizing scattering noise effect between the fan beams.

### 2. Background

X-ray computed tomography is used in various clinical fields such as diagnosis, real-time imaging during surgery, and postoperative prognosis evaluation, etc. Further, computed tomography is applied not only to medical diagnostic imaging devices, but also to the purpose of airport cargo inspection or nondestructive inspection of industrial products such as microstructures.

In a computed tomography apparatus, x-rays are projected onto a test subject, wherein some are absorbed in the test subject, and the remaining transmitted radiation are detected by a plurality of detectors arranged in a linear or planar shape, and then the output data of each detector is converted into electrical signals to reconstruct images, thereby obtaining a tomographic image of the test subject.

In a conventional computed tomography apparatus, x-ray projection data is obtained by rotating a gantry installed near the test subject, and thus there are disadvantages such as electricity supply to the gantry, real-time transmission of large-capacity data, and unease of precise position control. Further, it takes a long time for computed tomography to acquire tomography image data, and it takes a considerable amount of time to reconstruct the acquired projection data to obtain a three-dimensional stereoscopic image, which has been a technical obstacle to using computed tomography during surgery in real time. The KR 101 665 327 B1 discloses a CT device comprising a plurality of sources arranged on a circumference of a plurality of gantries, and detectors placed on the circumference of the gantries opposite to the sources.

### SUMMARY

Therefore, a purpose of the present disclosure is to resolve such problems of prior art, that is to provide a computed tomography apparatus where fan beam type multi-sources are spaced along a transfer direction of a test subject, with one pair of adjacent sources having an angle between 90 to 180 degrees, thereby minimizing scattering noise effect between the fan beams, and a manufacturing method and operating method thereof.

The aforementioned purpose is achieved by a computed tomography apparatus as defined in claim 1 and an operating method for a computed tomography apparatus as defined in claim 2.

### BRIEF DESCRIPTION OF THE DRAWINGS

FIG. 1 is a view illustrating a gantry constituting a computed tomography apparatus according to an embodiment of the present disclosure;
FIG. 2 is a view illustrating a planar structure of a computed tomography apparatus according to an embodiment of the present disclosure;
FIG. 3 is a view illustrating a front structure of a computed tomography apparatus according to an embodiment of the present disclosure;
FIG. 4 is a view illustrating a tomography switching operation sequence for each gantry according to the present disclosure;
FIG. 5 is a view illustrating an angle between a first source of a first gantry, a first source of a second gantry, and a first source of a third gantry of a computed tomography apparatus according to an embodiment of the present disclosure;
FIGs. 6 and 7 are views illustrating two embodiments of a sequence for operating a computed tomography apparatus according to an embodiment of the present disclosure;
FIG. 8 is a front configuration view of a computed tomography apparatus according to an embodiment of the present disclosure;
FIG. 9 is a three-dimensional view illustrating an arrangement relationship of N sources and detectors illustrated in FIG. 8; and
FIG. 10 is a view illustrating front views of the N sources and detectors illustrated in FIG. 9.

### *REFERENCE NUMERALS*

100: GANTRY, 110: SOURCE, 120: DETECTOR, 200: TEST SUBJECT, 300: TRANSFER UNIT

### DETAILED DESCRIPTION

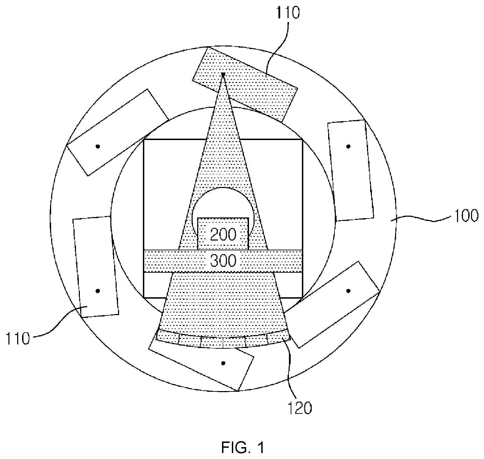

Hereinbelow, a computed tomography apparatus according to an embodiment of the present disclosure will be described in detail with reference to FIGs. 1 to 5.

FIG. 1 is a view illustrating a gantry constituting a computed tomography apparatus according to a preferred embodiment of the present disclosure. Referring to FIG. 1, a gantry 100 according to the present disclosure is characterized not to rotate, but to be stationary. Referring to FIG. 1, a test subject 200 is placed on top of a transfer unit 300, and under the control of a control apparatus, the transfer unit 300 transfers the test subject 200 in z direction. In the drawings, the plane on which the gantry 100 is placed is taken as an x-y plane for convenience.

The gantry 100 according to the present disclosure is characterized to have a plurality of sources 110 that irradiate a fan beam shape x-ray to the test subject 200. Referring to FIG. 1, as a preferred embodiment of the present disclosure, a total of six sources 110 are arranged at equal intervals on a circumference of the gantry. Further, on the opposite side of each source, a detector 120 corresponding to each source is installed, and as a preferred embodiment of the present disclosure, a total of six detectors 120 are arranged on the circumference of the gantry (illustration omitted). Here, according to a preferred embodiment of the present disclosure, the detector 120 may consist of a total of six photodetectors, but there is no limitation thereto.

As a preferred embodiment of the present disclosure, having a plurality of gantries to obtain a tomographic image is a technical characteristic. For convenience, the method according to the present disclosure may be referred to as a multi-gantry computed tomography apparatus.

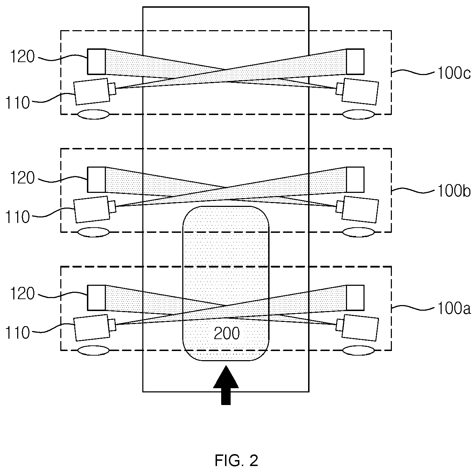

**FiG. 2** is a view illustrating a multi-gantry computed tomography apparatus according to the present disclosure. Referring to **FIG. 2****,** as an embodiment of the present disclosure, three gantries 100a, 100b, 100c are illustrated. A computed tomography apparatus according to an embodiment of the present disclosure consists of a total of three gantries 100a, 100b, 100c, and each of the gantries 100a, 100b, 100c has a total of six sources 110 and a total of six detectors 120. According to a preferred embodiment of the present disclosure, the test subject 200 is transferred in a z-axis direction by motor control in a state in which it is placed on top of the transfer unit 300, and a tomographic image of an x-y plane is obtained.

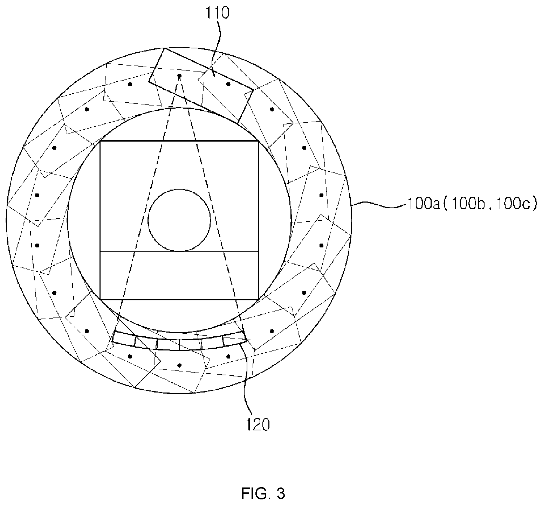

FIG. 3 is a view schematically showing the sources 110 attached to the multi-gantry computed tomography apparatus according to the present disclosure by overlapping the sources on one drawing. Since the sources are illustrated to overlap in one drawing, it should be noted that a total of eighteen sources 110 are illustrated. On each of the three gantries 100a, 100b, 100c, six sources 110 are arranged at intervals of 60 degrees, and each gantry 100a, 100b, 100c is arranged to stagger by 20 degrees, and thus, a total of eighteen sources 110 are installed on the circumference of the gantries each spaced apart by 20 degrees without overlapping each other. Further, on an opposite side of each source 110, the detector 120 corresponding to the corresponding source 110 is disposed to face the source 110.

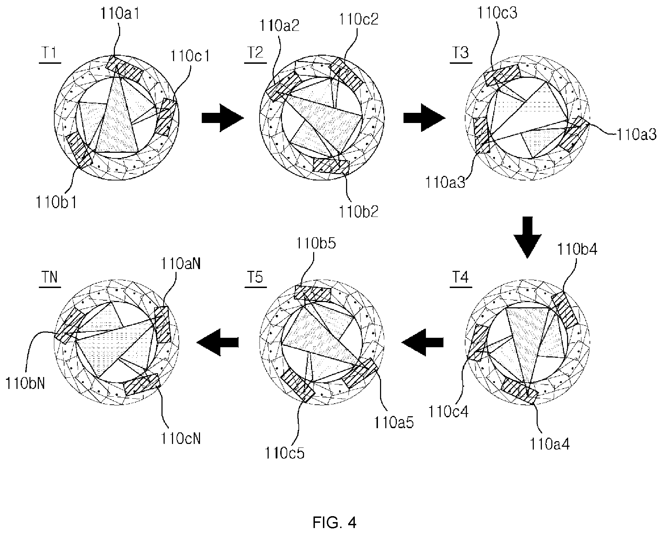

FIG. 4 is a view illustrating a tomography switching operation sequency per gantry according to a preferred embodiment of the present disclosure. Referring to FIG. 4, a first source 110a1, ..., an N^{th} source 110aN of a first gantry 100a; a first source 110b1, ..., an N^{th} source 110bN of a second gantry 100b; and a first source 110c1, ..., an N^{th} source 110cN of a third gantry 100c may each be designated according to an operating order, and the operating order is set such that the angle between the plurality of sources that operate sequentially is 90 to 180 degrees.

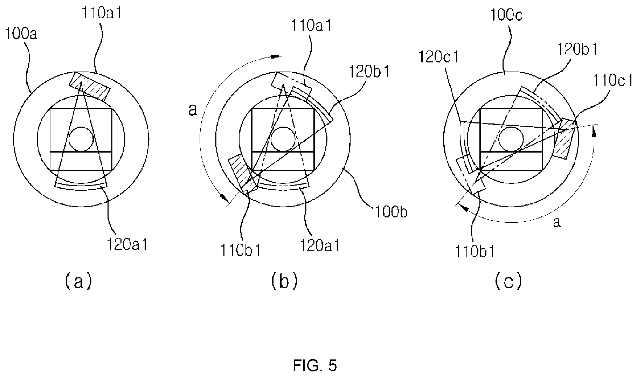

FIG. 4 illustrates an example where it is set such that the sources operating sequentially according to the operating order have an angle of 140 degrees between each other, and FIG. 5 illustrates a position relation of the source of each gantry and detector at T1 period, in which if the first source 110a1 of the first gantry, the first source 110b1 of the second gantry, and the first source 110c1 of the third gantry irradiate x-rays at the T1 period, the x-ray that passed through the test subject is detected at a first detector 120a1 of the first gantry, a first detector 120b1 of the second gantry, and a first detector 120c1 of the third gantry installed on the opposite side, thereby obtaining a tomographic image.

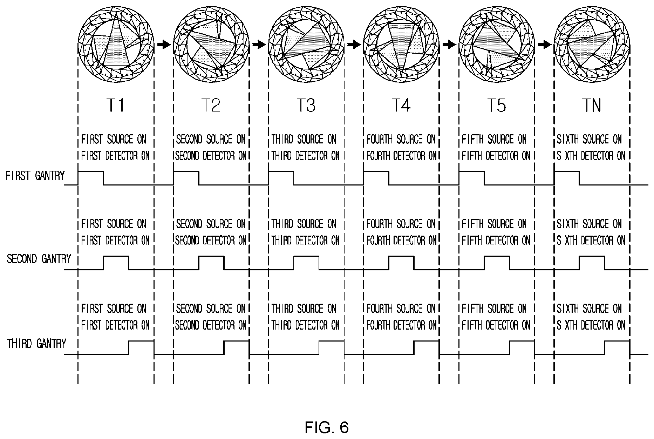

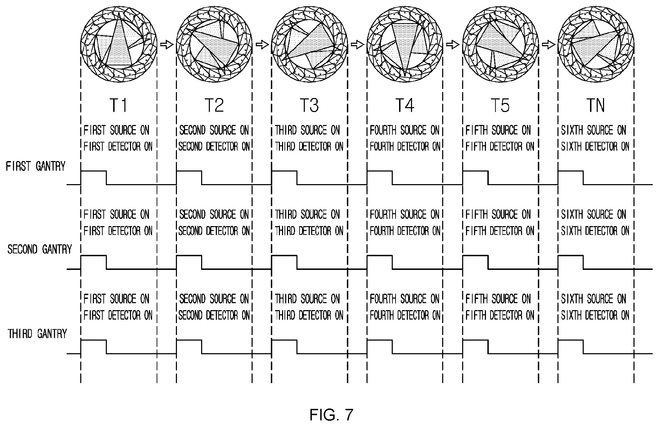

Here, according to a preferred embodiment of the present disclosure, if a total of L gantries are used, and a total of N sources and detectors are used in each gantry, the spaced angle between the sources installed in each gantry is 360/(L x N) degrees. Here, the timing of obtaining a tomographic image may operate in a normal mode of conducting sequentially at the T1 period (refer to FIG. 6), or operate in a high-speed mode of conducting simultaneously (refer to FIG. 7).

Referring to FIG. 4 again, at a T2 period, from a second source 110a2 of the first gantry, a second source 110b2 of the second gantry, and a second source 110c2 of the third gantry, an x-ray is irradiated to the test subject, and the second detector installed on the opposite side corresponding to the second source in each gantry, a transmission x-ray is detected and sent to a processor (illustration omitted), and converted into a tomographic image.

In the same method, at a TN period, from an N^{th} source 110aN of the first gantry, an N^{th} source 110bN of the second gantry, and an N^{th} source 110cN of the third gantry, an x-ray is irradiated to the test subject, and from an N^{th} detector installed on the opposite side corresponding to the N^{th} source in each gantry, a transmission x-ray is detected and sent to a processor (illustration omitted), and converted into a tomographic image.

Here, of the plurality of sources for photographing sequentially per each period, the angle between one pair of sources adjacent in the transfer direction (z direction) of the test subject is set to be within 90 to 180 degrees.

That is, at T1 period, as in (a) of FIG. 5, the fan beam type x-ray projected from the first source 110a1 of the first gantry 100a passes through the test subject to be detected at the first detector 120a1 of the first gantry 100a, and as in (b) of FIG. 5, the fan beam type of x-ray projected from the first source 110b1 of the second gantry 100b is detected at the first detector 120b1 of the second gantry 100b, and as in (c) of FIG. 5, the fan beam type x-ray projected from the first source 110c1 of the third gantry 100c is detected at the first detector 120c1 of the third gantry 100c. Here, as the angle a of the first source 110a1 of the first gantry 100a and the first source 110b1 of the second gantry 100b and the angle a of the first source 110b1 of the second gantry 100b and the first source 110c1 of the third gantry 100c are each set to be 90 to 180 degrees, the first detector 120a1 of the first gantry 100a and the first detector 120b1 of the second gantry 100b, and the first detector 120b1 of the second gantry 100b and the first detector 120c1 of the third gantry 100c, adjacent in z direction, come to be located in relatively opposite direction to each other, and thus it is possible to minimize the scatter noise effect between the fan beams, and accordingly, it is possible to not only avoid a direct over scan region, but also avoid occurrence of noise due to back scatter detection.

Meanwhile, since the first detector 120a1 of the first gantry 100a and the first detector 120c1 of the third gantry 100c are spaced apart from each other having the second gantry 100b between them, even if an arrangement direction of the first detector 120a1 of the first gantry 100a and the first detector 120c1 of the third gantry 100c is set to be relatively parallel, they may not be under the scatter noise effect between the fan beams.

FIGs. 6 and 7 are views illustrating two embodiments of a sequence for operating a multi-gantry/multi-source/multi-detector computed tomography apparatus according to the present disclosure. The sequence illustrated in FIG. 6 may be referred to as a sequential switching mode, and the sequence illustrated in FIG. 7 may be referred to as a high speed switching mode.

Referring to FIG. 6, at T1 period, from the first source of the first gantry, the first source of the second gantry and the first source of the third gantry, an x-ray is sequentially emitted, and photographing is conducted at the first detector of the first gantry, the first detector of the second gantry and the first detector of the third gantry, to obtain a tomographic image.

If the time it takes for an x-ray to be projected from a source and an image is obtained from the transmitted x-ray amount detected at the detector is △t, it is preferable that a signal that triggers the first source of each gantry is applied at a timing of △t/L. For example, if the time it takes for an x-ray to be projected from one source and an image is obtained from the transmitted x-ray amount detected at the detector △t is 20 to 25 msec, the time it takes to detect the data of the transmitted x-ray projected from all the sources constituting each gantry becomes T = N △t, and thus, the number of times per second 3D images can be obtained is f = 1/T = 6.6 ~ 8.3 Hz, that is, about 7 to 8 times.

Referring to FIG. 7, at T1 period, from the first source of the first gantry, the first source of the second gantry, and the first source of the third gantry, an x-ray is simultaneously emitted, and photographed to obtain a tomographic image. The time it takes for an x-ray to be projected from one source and an image is obtained from the transmitted x-ray amount detected at the detector △t is about 8 to 10 msec, and thus the time it takes for detecting the data of the transmitted x-ray from all the sources constituting each gantry is T = 6 △t, and thus the number of times per second 3D images can be obtained is f = 1/T = 16.6 ~ 20 Hz, that is, about 20 times.

A computed tomography apparatus according to the present disclosure is characterized to be equipped with a dual energy detector. The dual energy detector according to the present disclosure is characterized to primarily detect the transmitted ray amount coming out from a test subject after a high energy (for example 120 KeV) x-ray is projected onto the test subject and then to secondarily detect the same, thereby obtaining a high energy image and a low energy image. The x-ray source creates one wide spectrum or multiple peak x-ray spectrum, and the detector uses a filter to simultaneously obtain an image by energy band depending on the x-ray photon energy, thereby obtaining a dual energy image.

Hereinbelow, a computed tomograph apparatus according to an embodiment of the present disclosure will be described.

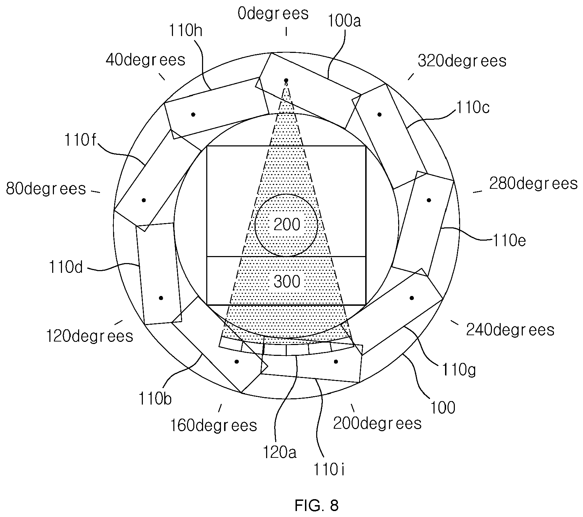

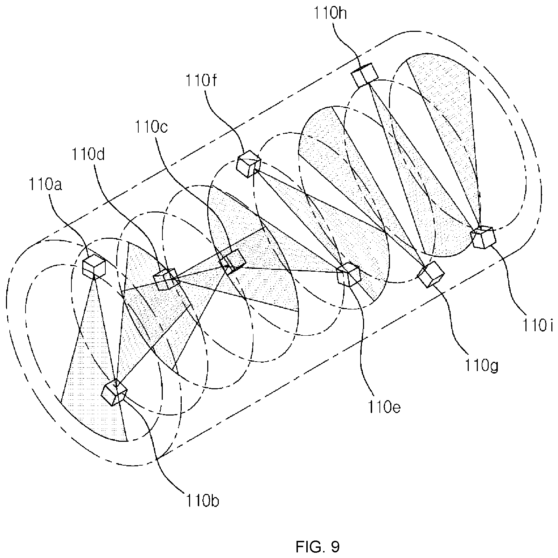

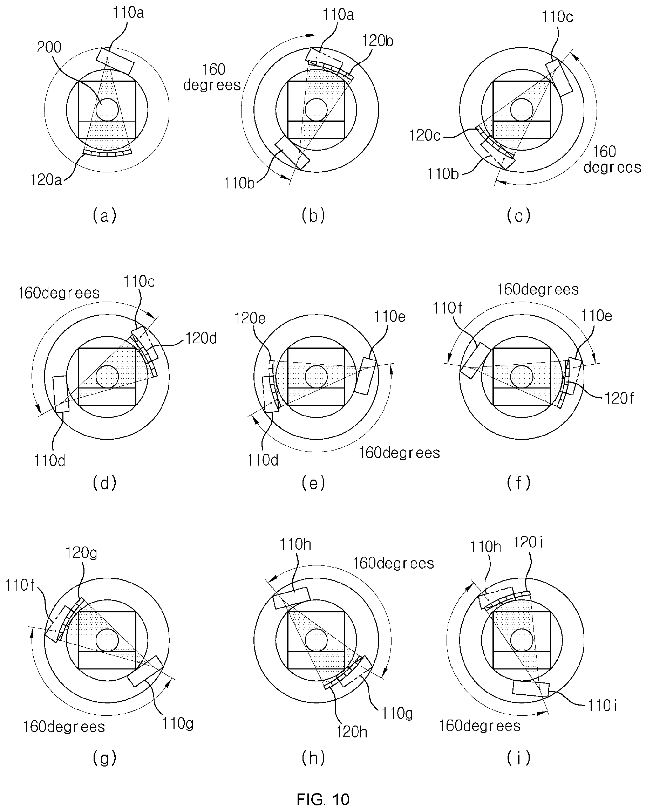

Of the attached drawings, FIG. 8 is a front configuration view of a computed tomography apparatus according to an embodiment of the present disclosure, FIG. 7 is a three-dimensional view illustrating an arrangement relationship of N sources and detectors illustrated in FIG. 8, and FIG. 10 is a view illustrating front views of the N sources and detectors illustrated in FIG. 9.

The computed tomography apparatus according to an embodiment of the present disclosure illustrated in FIGs. 8 to 10 includes a cylindrical gantry 100 that has a central axis arranged in z direction that is parallel with a transfer direction of a test subject and that is arranged to be stationary (not rotatable), N sources 110a to 110i that are spaced apart around the central axis of the cylindrical gantry 100 on the circumference at different angles and generate fan beam type x-rays towards the test subject 200 that passes through the center of the gantry 100, and a total of N detectors 120a to 120i arranged on the opposite side on the circumference to correspond to each of the sources 110a to 110i in order to detect the x-ray that is projected from the sources 110a to 110i and passed through the test subject.

The N sources 110a to 110i may consist of a total of nine sources, and the total of nine sources 110a to 110i may be arranged on the circumference of the gantry 100 to have intervals of 40 degrees when seen from the front as in FIG. 8. Further, the N detectors 120a to 120i may consist of a total of nine detectors just as the sources, and are arranged to face the sources 110a to 110i having the test subject 200 between them at the opposite side of the sources 110a to 110i on each circumference.

The test subject 200 is transferred in a z-axis direction by motor control in a state in which it is placed on top of the transfer unit 300, and a tomographic image of an x-y plane is obtained by the N sources 110a to 110i and detectors 120a to 120i.

As in FIGs. 9 and 10, the N sources 110a to 110i may be differentiated as first to nine sources 110a to 110i depending on the z direction arrangement order, and are set such that the angle between one pair of sources adjacent in z direction is within 90 to 180 degrees. The present embodiment is described based on an example where a total of nine sources 110a to 110i are set to be arranged at intervals of 40 degrees, and the angle between the one pair of sources adjacent in z direction is set to be 160 degrees.

Specifically, in a case of trying to photograph a tomograph of the test subject 200 while going around the test subject 200 using a total of nine sources 110a to 110i, since nine sources 110a to 110i must be arranged around the test subject 200 at very 40 degrees, a total of nine sources 110a to 110i may have arrangement angles of 0 degree, 40 degrees, 60 degrees, 120 degrees, 160 degrees, 200 degrees, 240 degrees, 280 degrees and 320 degrees.

Here, when the arrangement angle of the first source 110a is selected as 0 degree, the arrangement angle of the second source 110b may be selected to be 160 degrees which is any one of the degrees having 90 to 180 degrees of angle with respect to 0 degree: 120 degrees, 160 degrees, 200 degrees, and 240 degrees. Next, the arrangement angle of the third source 110c may be selected to be 320 degrees which is any one of the remaining degrees having an angle of 90 to 180 degrees with respect to 160 degrees: 280 degrees, 320 degrees, 0 degree and 40 degrees and excluding 0 degree; the arrangement angle of the fourth source 110d may be selected to be 120 degrees which is any one of the degrees having an angle of 90 to 180 degrees with respect to 320 degrees: 80 degrees, 120 degrees, 160 degrees and 200 degrees and excluding 160 degrees that is the arrangement angle of the second source 110b; and the arrangement angle of the fifth source 110e may be selected to be 280 degrees which is any one of the degrees having an angle of 90 to 180 degrees with respect to 120 degrees: 240 degrees, 280 degrees, 320 degrees and 0 degree and excluding 0 degree that is the arrangement angle of the first source 110a.
According to this principle, the arrangement angle of the sixth source 110f may be selected as 80 degrees, the arrangement angle of the seventh source 110g may be selected as 240 degrees, the arrangement angle of the eighth source 110h may be selected as 40 degrees, and the arrangement angle of the ninth source 110i is may be selected as 200 degrees.

That is, as described above, when the total of nine sources 110a to 110i are arranged at different angles to each other around the test subject 200, but the angle between a pair of adjacent sources is set to 90 to 180 degrees, a pair of adjacent detectors come to be located in opposite directions to each other, and thus the effect of scattering noise between fan beams can be minimized, thereby avoiding the direct over scan regions and reducing occurrence of noise caused by back scatter detection.

Further, unlike the prior art of continuously irradiating x-rays while rotating 360 degrees around the test subject 200, in the computed tomography apparatus according to the present embodiment, a total of N sources irradiate x-rays only from a plurality of fixed arrangement angles around the test subject, the amount of x-rays exposed to the test subject can be reduced.

Meanwhile, when arranging the arrangement angles of the plurality of sources 110a to 110i irregularly instead of sequentially as described above, in the process of displaying a plurality of tomographic images obtained using the plurality of sources 110a to 110i and detectors 120a to 120i, the tomographic images may appear to have different angles from each other on the display depending on the arrangement angle (photography angle) of the sources 110a to 110i, and thus it is preferable to compensate for the correction values corresponding to the arrangement angles of the sources 110a to 110i so that a plurality of tomographic images are displayed at the same angle as a whole.

Further, if the plurality of tomographic images obtained from the plurality of detectors are aligned in the order of the photographing angle, it is possible to obtain a plurality of tomographic images in the form of spirally rotating around the test subject, and thus not only can continuous volume data be obtained like a helical computed tomography apparatus (Helical CT), but it is also possible to convert it into a 3D image using a dedicated algorithm and provide it to the user.

In addition, in the process of moving the test subject 200 through the transfer unit 300, in obtaining the images in the order of arrangement of the sources 110a to 110i and detectors 120a to 120i, instead of waiting until all the tomographic images are obtained from the total of N detectors 120a to 120i, the tomographic images obtained from each detector 120a to 120i are preferentially displayed on the display, so that the reader can read ahead using the displayed tomographic images, thereby minimizing the reading delay time.

A computed tomography apparatus operating method according to the present disclosure includes transferring a test subject (S110), obtaining a tomographic image (S120), correcting a tomographic image inclination (S130), and displaying the tomographic image (S140).

At the transferring a test subject (S110), the test subject 200 is transferred in z direction using the transfer unit 300.

At the obtaining a tomographic image (S120), the first source to ninth source 110a to 110i that are arranged at different angles to each other on a circumference spaced apart in z direction and of which the angle between one pair of adjacent sources is set to 90 to 180 degrees are operated sequentially, and the x-ray transmitted through the test subject 200 is detected sequentially at the first detector to ninth detector 120a to 120i, to obtain a tomographic image of the test subject 200.

At the correcting a tomographic image inclination (S130), the inclination of the tomographic image according to the arrangement angle of the sources 110a to 110i is corrected such that the plurality of tomographic images obtained at the detectors 120a to 120i can be displayed at certain angles. For example, the tomographic image obtained at the first detector 120a may be set as a reference angle, and to the tomographic image obtained at the second detector 120b that has an angle of 160 degrees between the first detector 120a, a correction value of -160 degrees may be applied, so that the tomographic image obtained at the second detector 120b can be aligned at the same angle as the tomographic image obtained at the first detector 120a.

At the displaying the tomographic image (S140), the tomographic images obtained at the total of N detectors 120a to 120i are displayed on a display (not illustrated) in the order obtained. That is, in the process where the test subject 200 passes through the gantry 100, when the tomographic images obtained at the detectors 120a to 120i are displayed on the display immediately instead of waiting until all the tomographic images are obtained at the total of N detectors 120a to 120i, the reader can read ahead in the scanning process, and thus reading delay time can be minimized.

Meanwhile, after the total of N tomographic images are all obtained, a step of aligning the N tomographic images in the order of photographing angle, and a step of creating 3D image data using the aligned N 2D tomographic images may be further performed.

For example, the tomographic images obtained by the total of nine sources 110a to 110i and detectors 120a to 120i are photographed in the order of 0 degree, 160 degrees, 320 degrees, 120 degrees, 280 degrees, 80 degrees, 240 degrees, 40 degrees and 200 degrees based on the arrangement angles of the sources 120a to 120i. Therefore, when these are aligned in the order of arrangement angles, it is possible to obtain tomographic images in the form of spirally rotating around the test subject 200, which can be converted it into 3D image data using a spiral computed tomography apparatus algorithm and provide it to the user.

In addition, a computed tomography apparatus operating method according to the present disclosure may include, in a utilizing method of data obtained from a total of N source-detector system generating a fan beam type dual energy peak x-rays, (a) exactly correcting a volume calculation of a material from images photographed at N angles; and (b) calculating absorbance of the material individually in dual energy images photographed at N angles; and (c) calculating density based on the absorbance and volume of the material; and (d) determining components of the material using absorbance and density; and (e) realizing a tomographic image based on the components of the material, and a morphological tomographic image and a tomographic image by material component can be obtained together.

The scope of the present disclosure is not limited to the above-described embodiments, but may be implemented in various forms of embodiments within the scope of the appended claims. Anyone skilled in the art without departing from the subject matter of the present disclosure claimed in the claims is considered to be within the scope of the claims of the present disclosure to various extents that can be modified.

## Claims

1. A computed tomography apparatus comprising: a plurality of stationary gantries L (100); N sources that are arranged on each of the stationary gantries at different angles to each other one by one on a plurality of circumferences arranged to overlap along a transfer direction, z-direction, of a test subject and that generate a fan beam type x-ray towards the test subject; and
N detectors that are arranged on opposite sides on the circumference of each of the gantries corresponding to each source and that detect the x-rays that are transmitted through the test subject,
wherein the N sources are each arranged at N arrangement angles with a spaced angle of 360/N degrees, and the total of the sources are installed on the circumference of the gantries each spaced apart by 360/(NxL) degrees,
wherein the N sources (110) are configured to operate sequentially, so that at each time period T_{N} an x-ray is irradiated from the Nth source of each of the gantries and detected by an Nth detector installed on the opposite side corresponding to the Nth source in each gantry, **characterized in that** the angle between the sources (110) operating on adjacent gantries (100) at each time period T_{N} is within the range of 90 to 180 degrees.

2. An operating method of a computed tomography apparatus according to claim 1, comprising:
transferring the test subject to penetrate a center of the circumference; and
operating the sources sequentially as indicated in claim 1 to obtain a tomographic image of the test subject.

3. The operating method of a computed tomography apparatus according to claim 2, comprising:
displaying the tomographic image obtained at the detector on a display in the order obtained.

4. The operating method of a computed tomography apparatus according to claim 3, comprising:
correcting an inclination of the tomographic image according to the arrangement angle of the source, such that N tomographic images obtained at the detector can be displayed at certain angles, before the tomographic image is displayed on the display.

5. The operating method of a computed tomography apparatus according to claim 2, further comprising, after obtaining all the N tomographic images:
aligning the N tomographic images in the order of photographing angle; and
creating a three dimensional image data using N aligned two dimensional tomographic images.

## Patentansprüche

1. Computertomographievorrichtung, umfassend: mehrere stationäre Gantrys L (100); N Quellen, die auf jedem der stationären Gantrys in unterschiedlichen Winkeln zueinander auf mehreren Umfangslinien angeordnet sind, die so angeordnet sind, dass sie sich entlang einer Transferrichtung, der z-Richtung, eines Prüflings überlappen, und die einen fächerförmigen Röntgenstrahl in Richtung des Prüflings erzeugen; und
N Detektoren, die auf gegenüberliegenden Seiten auf dem Umfang jedes der Gantries entsprechend jeder Quelle angeordnet sind und die die durch den Prüfling übertragenen Röntgenstrahlen erfassen,
wobei die N Quellen jeweils in N Anordnungswinkeln mit einem Abstandwinkel von 360/N Grad angeordnet sind, und die Summe der Quellen auf dem Umfang der Gantries jeweils um 360/(NxL) Grad beabstandet installiert sind,
wobei die N Quellen (110) so konfiguriert sind, dass sie sequenziell arbeiten, so dass in jedem Zeitraum T_{N} ein Röntgenstrahl von der N-ten Quelle jedes der Gantries ausgestrahlt und von einem N-ten Detektor erfasst wird, der auf der gegenüberliegenden Seite entsprechend der N-ten Quelle in jedem Gantry installiert ist, **dadurch gekennzeichnet, dass** der Winkel zwischen den Quellen (110), die in benachbarten Gantries (100) in jedem Zeitraum T_{N} arbeiten, im Bereich von 90 bis 180 Grad liegt.

2. Verfahren zum Betreiben einer Computertomographievorrichtung nach Anspruch 1, umfassend:
Bewegen des Prüflings, so dass er einen Mittelpunkt des Umfangs durchdringt; und
Betreiben der Quellen nacheinander, wie in Anspruch 1 angegeben , um ein tomographisches Bild des Prüflings zu erhalten.

3. Verfahren zum Betreiben einer Computertomographievorrichtung nach Anspruch 2, umfassend:
Anzeigen des am Detektor erhaltenen tomographischen Bildes auf einer Anzeige in der Reihenfolge, in der sie erhalten wurden.

4. Verfahren zum Betreiben einer Computertomographievorrichtung nach Anspruch 3, umfassend:
Korrektur einer Neigung des tomographischen Bildes entsprechend dem Anordnungswinkel der Quelle, so dass N am Detektor erhaltene tomographische Bilder in bestimmten Winkeln angezeigt werden können, bevor das tomographische Bild auf der Anzeige angezeigt wird.

5. Verfahren zum Betreiben einer Computertomographievorrichtung nach Anspruch 2, ferner umfassend, nachdem alle N tomographischen Bilder erhalten wurden:
Ausrichten der N tomographischen Bilder in der Reihenfolge der Aufnahmewinkel; und
Erzeugen von dreidimensionalen Bilddaten unter Verwendung von N ausgerichteten zweidimensionalen tomographischen Bildern.

## Revendications

1. Appareil de tomographie par ordinateur comprenant : une pluralité de portiques fixes L (100) ; N sources qui sont disposées sur chacun des portiques fixes à des angles différents les unes par rapport aux autres, une par une, sur une pluralité de circonférences disposées de manière à se chevaucher le long d'une direction de transfert, la direction z, d'un sujet testé et qui génèrent un faisceau de rayons X de type en éventail vers le sujet testé ; et
N détecteurs qui sont disposés sur des faces opposées sur la circonférence de chacun des portiques correspondant à chaque source et qui détectent les rayons X qui sont transmis à travers le sujet testé,
dans lequel les N sources sont chacune disposées à N angles de disposition avec un angle espacé de 360/N degrés, et le total des sources étant installé sur la circonférence des portiques espacés les uns des autres de 360/(NxL) degrés,
dans lequel les N sources (110) sont configurées pour fonctionner séquentiellement, de sorte qu'à chaque période de temps TN, un rayon X est émis par la N source de chacun des portiques et détecté par un N détecteur installé sur le côté opposé correspondant à la N source dans chaque portique, **caractérisé en ce que** l'angle entre les sources (110) fonctionnant sur des portiques adjacents (100) à chaque période de temps TN est compris dans la plage de 90 à 180 degrés.

2. Méthode opérationnelle d'un appareil de tomographie par ordinateur selon la revendication 1, comprenant :
transférer le sujet testé pour qu'il traverse un centre de la circonférence ; et
opérer les sources de manière séquentielle comme indiqué dans la revendication 1 pour obtenir une image tomographique du sujet testé.

3. Méthode opérationnelle d'un appareil de tomographie par ordinateur selon la revendication 2, comprenant :
affichage de l'image tomographique obtenue au niveau du détecteur sur un écran dans l'ordre obtenu.

4. Méthode opérationnelle d'un appareil de tomographie par ordinateur selon la revendication 3, comprenant :
la correction d'une inclinaison de l'image tomographique en fonction de l'angle d'agencement de la source, de telle sorte que N images tomographiques obtenues au niveau du détecteur puissent être affichées à certains angles, avant que l'image tomographique ne soit affichée sur l'écran.

5. Méthode opérationnelle d'un appareil de tomographie par ordinateur selon la revendication 2, comprenant en plus, après avoir obtenu toutes les N images tomographiques :
l'alignement des N images tomographiques dans l'ordre de l'angle de photographie ; et
la création de dates d'image tridimensionnelles en utilisant N images tomographiques bidimensionnelles alignées.
